# FASCICULE DE BREVET EUROPEEN

(11) **EP 3 294 868 B1**
(45) Date de publication et mention de la délivrance du brevet: **02.03.2022**
(21) Numéro de dépôt: 16729015.4
(22) Date de dépôt: 11.05.2016
(51) Int. Cl.: C12N 1/20

(54) **ENRICHISSEMENT ET CULTURE SELECTIVE DE SALMONELLA ET DE SHIGELLA**
ANREICHERUNG UND SELEKTIVE KULTUR VON SALMONELLA UND SHIGELLA
ENRICHMENT AND SELECTIVE CULTURE OF SALMONELLA AND SHIGELLA

(30) Priorité: 12.05.2015 FR 1554223
(43) Date de publication de la demande: 21.03.2018
(73) Titulaire: Biomérieux, 69280 Marcy l'Étoile (FR)
(72) Inventeur: BASSO, Emanuelle, 69770 Montrottier (FR); ROCHE, Jean-Marc, 73350 Feissons sur Salins (FR)
(86) Numéro de dépôt international: PCT/FR2016/051102
(87) Numéro de publication internationale: WO 2016/181070

(56) Documents cités:
- "Ornithine Decarboxylase Broth", Himedia - Technical Data , janvier 2011 (2011-01), XP002752826, Extrait de l'Internet: URL:http://himedialabs.com/TD/M1223.pdf [extrait le 2016-01-11]
- "Decarboxylase Differential Media", Difco&BBL Manual, 2nd edition. , 2009, XP002752827, Extrait de l'Internet: URL:https://bd.com/europe/regulatory/Asset s/IFU/Difco_BBL/211430.pdf [extrait le 2016-01-11]
- Maria P. MacWilliams: "Indole Test Protocol", , 1 avril 2013 (2013-04-01), XP002752828, Extrait de l'Internet: URL:http://www.microbelibrary.org/componen t/resource/laboratory-test/3202-indole-tes t-protocol [extrait le 2016-01-11]
- Anonymous: "Moeller Decarboxylase Broth with Ornithine HCl (DM468)", Micromaster , 1 août 2008 (2008-08-01), pages 1-4, XP002752829, Extrait de l'Internet: URL:http://www.dehydratedculturemedia.co.i n/uploads/DM468-Moeller%20Decarboxylase%20 Broth%20with%20Ornithine%20HCL-U.pdf [extrait le 2016-01-12]
- ARROYO G ET AL: "Selective action of inhibitors used in different culture media on the competitive microflora of Salmonella", JOURNAL OF APPLIED BACTERIOLOGY, BLACKWELL PUBLISHING LTD., OXFORD, GB, vol. 78, no. 3, 1 janvier 1995 (1995-01-01), pages 281-289, XP002555076, ISSN: 0021-8847

## Description

La présente invention a trait à la détection et l'identification de bactéries appartenant aux genres *Salmonella* et *Shigella.* Plus précisément, elle concerne les méthodes de microbiologie et les milieux de culture utilisés pour la détection, l'identification, l'isolement et/ou l'étude analytique de ces bactéries.

Appartenant à la famille des *Enterobacteriaceae,* les salmonelles et les shigelles sont des bactéries pathogènes pour l'homme. Transmissibles à l'homme après ingestion d'eau et/ou d'aliments souillés par des selles d'origine animale ou humaine, elles sont à l'origine de maladies et de troubles digestifs pouvant conduire à des séquelles irréversibles, parfois létales.

S'agissant des salmonelles, ces bactéries se transmettent généralement à l'homme par ingestion d'eau et/ou d'aliments souillés par des fécès animales.

A l'exception de *Salmonella typhi* et *Salmonella paratyphi,* les salmonelles sont responsables de la salmonellose, une infection inflammatoire du tube digestif qui provoque crampes abdominales, diarrhée, fièvre et nausée. Ces symptômes surviennent très rapidement après la contamination (généralement, après 8 à 72 heures d'incubation). Sans traitement particulier, la rémission survient généralement au bout de quatre à sept jours. Pour les personnes à risque (en particulier, les patients immunodéprimés, les nourrissons, les personnes âgées, les femmes enceintes), un traitement antibiotique est préconisé pour éviter toute complication grave, voire létale : diarrhée et déshydratation sévères nécessitant une hospitalisation, hémorragie digestive, perforation intestinale, pneumonie, infection aiguë de la vésicule biliaire, hépatite, septicémie pouvant aboutir à une infection généralisée (os, cœur, cerveau, reins...).

Beaucoup plus virulentes, les espèces *Salmonella* Typhi et *Salmonella* Paratyphi sont responsables des fièvres typhoïdes et paratyphoïdes. Ces bactéries se développent d'abord au niveau des ganglions lymphatiques intestinaux du sujet, puis traversent la paroi intestinale avant d'atteindre la circulation sanguine où elles libèrent leurs toxines. Une fièvre continue apparaît alors et ce, généralement au bout de une à trois semaines après la contamination. Cette fièvre s'accompagne de maux de tête, d'anorexie, d'abattement, de douleurs abdominales et de diarrhées ou de constipations. Dans les formes bénignes, l'état du sujet reste stationnaire pendant une quinzaine de jours avant de laisser place à une convalescence de plusieurs semaines. Dans les formes plus graves, des complications surviennent au niveau de l'intestin grêle (où des performations peuvent survenir) et peuvent atteindre la vessie, le cœur, les articulations, le système nerveux... L'infection peut être fatale si le sujet n'a pas recours rapidement à un traitement antibiotique adéquat.

S'agissant des shigelles, ces bactéries se transmettent généralement à l'homme par ingestion d'eau et/ou d'aliments souillés par des selles humaines (issues de porteurs chroniques ou de malades atteints de shigelloses). Elles sont responsables des shigelloses, des dysenteries bacillaires causées par une infection inflammatoire du gros intestin.

A la différence d'autres maladies diarrhéiques moins sévères, la guérison du sujet n'est pas spontanée et nécessite plus qu'une réhydratation. Un traitement thérapeutique aux antibiotiques permet dans bien des cas une rémission rapide, sans séquelle.

Parmi les shigelles, *Shigella dysenteriae* apparait comme étant l'espèce la plus virulente. Elle est la seule à libérer des exotoxines. Elle provoque la destruction des tissus infectés et des diarrhées aigües, glaireuses et sanguinolentes.

Chaque année, les shigelloses tueraient entre six cent mille et un million de personnes dans le monde, pour l'essentiel des enfants de moins de 5 ans.

D'un point de vue clinique, le diagnostic précoce des infections par les salmonelles et les shigelles revêt une importance substantielle, et permettrait de proposer rapidement une solution thérapeutique adaptée aux patients.

Les premiers symptômes associés à une infection par ces bactéries se confondent malheureusement avec ceux d'une simple indigestion alimentaire ou d'un banal trouble gastro-intestinal, diarrhéique (en l'occurrence crampes abdominales, nausées, vomissement, fièvre, diarrhée). Un examen bactériologique des selles (EBS) est ainsi prescrit par le praticien dès lors que la présence d'un germe dangereux pour la santé du patient est suspecté.

A cet égard, plusieurs milieux de culture propices au développement et à la croissance aussi bien de *Salmonella* que de *Shigella,* sont connus. A titre d'exemple, on peut citer, les milieux du type gélose BCP (« *BromCresol Purple* »), les géloses aux désoxycholate, la gélose Hektoen, le bouillon « *GN Broth* » (bouillon selon Hajna), la Gélose *Salmonella-Shigella* (ou Gélose SS), la gélose MacConkey n°3, la gélose XLD (pour « *Xylose Lysine Desoxycholate* »), la gélose DCLS (« *Desoxycholate Citrate lactose Sucrose Agar »).*

La composition et la formulation de ces milieux sont décrites notamment dans le HANDBOOK OF MICROBIOLOGICAL MEDIA (2010 ; 4ème Edition).

Ces milieux permettent de répondre aux besoins nutritionnels des microorganismes à cultiver. Schématiquement, on trouve dans leur composition :
- de l'eau (généralement de l'eau distillée ou déionisée) ;
- au moins un glucide, à titre de source carbonée et d'énergie ; il s'agit généralement du glucose (éventuellement sous forme de saccharose ou de salicine) et/ou du lactose ;
- ainsi que d'autres éléments nutritifs (notamment, acides aminés, facteurs de croissance, vitamines, minéraux, oligo-éléments, citrate de fer, citrate de sodium, chlorure de sodium...) apportés sous la forme de compositions chimiquement complexes telles que des mélanges de peptones (de lait, de viande et/ou de fécules de pomme de terre et/ou de maïs...), d'extraits de levures, de sérum, et/ou des extraits de tissus d'origine animale et/ou végétale...
- également divers sels, tels que par exemple le chlorure de sodium, permettant d'établir une osmolarité adéquate au milieu.

Egalement, ces milieux peuvent présenter une certaine sélectivité à l'égard des salmonelles et des shigelles, grâce à l'emploi d'agents sélectifs inhibant au moins en partie la flore accompagnatrice (c'est-à-dire des bactéries autres que les salmonelles et les shigelles). A ce titre, en vue d'inhiber les bactéries à gram positif, sont classiquement utilisés :
- le désoxycholate de sodium,
- les sels biliaires,
- le violet cristal, et
- le vert brillant.

Ces milieux sont également, pour certains, discriminatifs et renferment, à cet effet, une composante chromogénique et/ou fluorogénique permettant une détection, éventuellement une identification, visuelles des microorganismes selon les activités métaboliques particulières qu'elles expriment. Il peut s'agir, par exemple :
- d'indicateurs colorés de pH (par exemple, le rouge neutre, le rouge de phénol, le pourpre de bromocrésol, le couple bleu de bromothymol et fuchsine acide) qui permettent de marquer par une coloration distinctive les colonies de microorganismes fermentaires d'au moins un des glucides présents dans le milieu (par exemple le glucose, le saccharose, le lactose et/ou la salicine) des autres bactéries ;
- d'un système thiosulfate de sodium / citrate de fer III, qui permet la mise en évidence des bactéries productrices de H₂S (en l'occurrence, les salmonelles), ces dernières apparaissant alors sous la forme de colonies à centre noir,
- de substrats chromogènes et/ou fluorogènes permettant la mise en évidence d'activités enzymatiques particulières.

Malgré cette grande diversité, les milieux de culture actuellement disponibles souffrent tous d'une sensibilité et d'une sélectivité relativement faibles, lorsqu'ils sont utilisés dans le cadre d'un examen bactériologique des selles, à des fins d'enrichissement et de recherche simultanée de *Salmonella* et de *Shigella.*

Ces inconvénients peuvent être expliqués essentiellement par la présence d'une flore microbienne intestinale/entérique extrêmement abondante et variée (des millions de microorganismes par gramme de selle, couvrant de nombreuses espèces bactériennes et fongiques), au milieu de laquelle, salmonelles et shigelles se trouvent en net sous-nombre. Une fois ensemencées sur/dans les milieux de culture traditionnels, les salmonelles et les shigelles, éventuellement présentes dans l'échantillon à tester, se retrouvent non seulement occultées physiquement par cette flore accompagnatrice, mais cette dernière interfère également avec leur croissance et leur développement, retardant d'autant plus leur détection.

La détection et l'identification des salmonelles et des shigelles, sur ces milieux de culture, est aussi rendue difficile par une grande ressemblance métabolique et physiologique avec d'autres bactéries intestinales.

Ainsi, en dépit de la grande variété de milieux de culture actuellement proposés pour la recherche de *Salmonella* et de *Shigella* dans les selles, il reste un besoin d'améliorer davantage leur sensibilité et leur sélectivité, et de raccourcir autant que possible le temps nécessaire à la détection. La présente demande vise à répondre à ces besoins.

Plus généralement, la présente invention vise à améliorer les méthodes et techniques utilisées pour détecter, identifier et/ou isoler les bactéries appartenant aux genres *Salmonella* et *Shigella.* Egalement, elle vise à proposer de nouvelles compositions de milieu de culture et/ou de bouillon d'enrichissement, aptes à permettre la culture et la multiplication des bactéries appartenant aux genres *Salmonella* et *Shigella,* et ayant des performances de sélectivité améliorées.

Avant de présenter l'invention, les définitions suivantes sont données pour permettre une meilleure compréhension de l'invention.

Par « milieu de culture », on entend un milieu comprenant tous les éléments nécessaires à l'expression d'un métabolisme et/ou à la croissance de microorganismes. Le milieu de culture peut être liquide, solide, semi-solide. Par « milieu solide » ou « milieu semi-solide », on entend par exemple un milieu gélifié. L'agar est l'agent gélifiant traditionnel en microbiologie pour la culture des microorganismes, mais il est possible d'utiliser de la gélatine, de l'agarose ou d'autres gélifiants naturels ou artificiels. Le caractère « solide » ou « semi-solide » des milieux dépend essentiellement de la teneur en agent gélifiant. A des fins de simplification, on utilisera par la suite l'expression « milieu solide » pour désigner aussi bien un milieu solide qu'un milieu semi-solide.

Par « bouillon d'enrichissement », on désigne plus spécifiquement un milieu de culture liquide.

Un milieu de culture est qualifié de milieu minimum, lorsque sa composition comporte uniquement les éléments chimiques strictement nécessaires à la croissance et la multiplication des microorganismes à cultiver. On y trouve classiquement :
- de l'eau (généralement, de l'eau distillée ou désionisée);
- une composante nutritive comprenant généralement :
   - une source carbonée et d'énergie (généralement du glucose et/ou du lactose) ;
   - une source de calcium (par exemple CaCl₂);
   - une source d'azote (par exemple (NH₄)₂SO₄) ;
   - une source de soufre (par exemple (NH₄)₂SO₄);
   - une source de magnésium (par exemple MgCl₂) ;
   - une source de fer (par exemple du citrate de fer) ;
   - une source d'oligo-éléments (par exemple sels de Cu, Zn, Co, Ni, B, Ti) ;
- éventuellement un tampon pH pour maintenir le milieu à un pH approprié ;
- éventuellement divers sels, à un niveau de concentration adapté pour conférer au milieu une osmolarité appropriée ; et
pour les milieux solides, éventuellement un agent gélifiant (par exemple l'agar, la gélatine ou l'agarose).

L'adjonction de facteurs de croissance particuliers et/ou de nutriments divers permet de renforcer les attributs de la composante nutritive et d'accroitre le pouvoir fertile du milieu. On parle alors de milieu « enrichi ». L'adjonction de ces facteurs de croissance et/ou de ces nutriments peut être réalisée au moyen de composés ou de compositions chimiquement définis, ou alors au moyen de compositions complexes (par exemple, sang frais, sérums, extrait de levure, peptones...).

Un milieu de culture est dit « sélectif » lorsqu'il comprend au moins un agent sélectif permettant audit milieu de favoriser la croissance d'un microorganisme-cible ou d'un groupe-cible de microorganismes, plutôt que celle de la flore accompagnatrice. Il s'agit essentiellement de composés aux effets antibiotiques et/ou antifongiques, et présentant une spécificité de toxicité (toxicité plus faible pour les microorganismes-cibles que pour la flore accompagnatrice).

Par « échantillon biologique », on entend un échantillon clinique issu d'un prélèvement d'origine humaine ou animale (en particulier, un échantillon de selle), ou un échantillon alimentaire issu de tout type d'aliment (par exemple, viande, œuf, légumes, mayonnaise, fromage, poisson, des boissons telles que le lait, un jus de fruits, l'eau...). Cet échantillon biologique peut être liquide ou solide. A des fins de simplification de vocabulaire, on utilisera indifféremment les expressions « échantillon » et « prélèvement ».

La présente invention, telle que définie par la revendication 1, concerne ainsi un procédé (une méthode) d'enrichissement et de culture sélective de bactéries appartenant aux genres *Salmonella* et/ou *Shigella* contenues dans un échantillon biologique, dans lequel on ensemence tout ou partie dudit échantillon dans/sur un milieu de culture comprenant une composante nutritive propice au développement et à la croissance desdites bactéries, caractérisé en ce que ledit milieu de culture comprend également de la L-ornithine à une concentration comprise entre 15 g/L et 20 g/L, à titre d'agent sélectif.

La présente invention repose sur le principe général d'incorporer, dans des compositions de milieux de culture envisagés pour la culture de *Salmonella* et/ou de *Shigella,* de la L-ornithine pour en accroitre la sélectivité à l'égard de ces bactéries et ce, au dépend de la flore accompagnatrice, qui se trouve au moins en partie inhibée. A cet effet, la L-ornithine est utilisée à une concentration comprise entre 15 g/L et 20 g/L.

Selon un mode préféré de mise en œuvre de l'invention, ledit milieu de culture comprend en outre, au moins un agent sélectif additionnel choisi parmi :
- le désoxycholate de sodium, notamment à une concentration comprise entre 0,1 g/L et 20 g/L, et plus préférentiellement encore entre de l'ordre de 1 g/L et de l'ordre de 10 g/L,
- le tergitol 4, notamment à une concentration comprise entre 0,5 mL/L et 10 mL/L, et plus préférentiellement encore entre de l'ordre de 1 mL/L et de l'ordre de 5 mL/L,
- des sels biliaires, notamment à une concentration comprise entre 0,1 g/L et 10 g/L, et plus préférentiellement encore entre de l'ordre de 0,5 g/L et 5 g/L,
- le violet cristal, notamment à une concentration comprise entre 0,05 mg/L et 50 mg/L, et plus préférentiellement encore entre de l'ordre de 0,1 mg/L et de l'ordre de 10 mg/L,
- le vert brillant, notamment à une concentration comprise entre 1 mg/L et 200 mg/L, et plus préférentiellement encore entre 10 mg/L et 50 mg/L.

Avantageusement et selon l'invention, ledit milieu de culture comprend en outre, à titre d'agents sélectifs :
- de la L-ornithine, à une concentration comprise entre 15 g/L et 20 g/L,
- du désoxycholate de sodium, à une concentration comprise entre de l'ordre de 1 g/L et de l'ordre de 10 g/L, et
- éventuellement, du tergitol 4, à une concentration comprise entre de l'ordre de 0,5 mL/L et de l'ordre de 5 mL/L.

Selon un mode avantageux de mise en œuvre de l'invention, ledit milieu de culture est un milieu discriminatif. En particulier, celui-ci comprend au moins une composante chromogénique choisie parmi :
- un indicateur coloré de pH (par exemple, le rouge neutre, le rouge de phénol, le pourpre de bromocrésol, le couple bleu de bromothymol et fuchsine acide) apte à réaliser un marquage des colonies de microorganismes fermentant au moins un des glucides présents dans le milieu ;
- un système thiosulfate de sodium / citrate de fer III, permettant la mise en évidence des bactéries productrices de H₂S ;
- des substrats chromogènes et/ou fluorogènes permettant la mise en évidence d'activités enzymatiques particulières.

Selon un aspect particulier, la présente invention vise à améliorer les méthodes de culture et/ou d'isolement des bactéries appartenant aux genres *Salmonella* et/ou *Shigella* qui ont actuellement cours, en intervenant sur la composition des milieux de culture usuels pour en accroitre la sélectivité en inhibant la flore accompagnatrice. A cet effet, la présente invention propose de parfaire la composition de ces milieux de culture, par un apport de L-ornithine, à une concentration finale comprise entre 15 g/L et 20 g/L, et, éventuellement, par un apport d'autres agents sélectifs comme précédemment énoncés.

Dans ce contexte, le procédé d'enrichissement et de culture sélective selon l'invention est avantageusement mis en œuvre avec un milieu de culture dont la composante nutritive reprend celle d'un milieu de culture choisi parmi :
- la Gélose XLD (bioMérieux, France),
- la Gélose chromID^{®} *Salmonella* Elite (bioMérieux, France),
- la Gélose *Salmonella-Shigella* (bioMérieux, France).

Selon un mode encore plus préféré, le procédé d'enrichissement et de culture sélective selon l'invention est mis en œuvre avec un milieu de culture comprenant une composante nutritive et une composante sélective reprenant celles de la Gélose XLD, et complémenté avec :
- de la L-ornithine, à une concentration comprise entre 15 g/L et 20 g/L,
- éventuellement, le X-*beta*-ribofuranoside, à une concentration comprise entre de l'ordre de 10 mg/L et de l'ordre de 200 mg/L,
- éventuellement, un agent opacifiant tel que le dioxide de titane (TiO₂), notamment à une concentration comprise entre de l'ordre de 0,1 g/L et de l'ordre de 5 g/L,
- éventuellement, du tergitol 4, à une concentration comprise entre de l'ordre de 0,5 mL/L et de l'ordre de 5 mL/L.

La présente invention s'étend également à un milieu de culture adapté à la mise en œuvre d'un procédé d'enrichissement et de culture sélective de bactéries appartenant aux genres *Salmonella* et *Shigella.* En l'espèce, un milieu de culture selon l'invention comprend une composante nutritive propice au développement et à la croissance desdites bactéries, et est caractérisé en ce qu'il comprend également de la L-ornithine, à titre d'agent sélectif.

Avantageusement et selon l'invention, ledit milieu de culture comprend une composante nutritive propice au développement et à la croissance des bactéries appartenant aux genres *Salmonella* et *Shigella,* et de la L-ornithine, à titre d'agent sélectif, et se caractérise également par tout ou partie des caractéristiques techniques suivantes :
- la concentration en L-ornithine est comprise entre 15 g/L et 20 g/L ;
- ledit milieu de culture comprend en outre, au moins un agent sélectif additionnel choisi parmi :
   - le désoxycholate de sodium, notamment à une concentration comprise entre 0,1 g/L et 20 g/L, et plus préférentiellement encore entre de l'ordre de 1 g/L et de l'ordre de 10 g/L,
   - le tergitol 4, notamment à une concentration comprise entre 0,5 mL/L et 10 mL/L, et plus préférentiellement encore entre de l'ordre de 1 mL/L et de l'ordre de 5 mL/L,
   - des sels biliaires, notamment à une concentration comprise entre 0,1 g/L et 10 g/L, et plus préférentiellement encore entre de l'ordre de 0,5 g/L et 5 g/L,
   - le violet cristal, notamment à une concentration comprise entre 0,05 mg/L et 50 mg/L, et plus préférentiellement encore entre de l'ordre de 0,1 mg/L et de l'ordre de 10 mg/L, et
   - le vert brillant, notamment à une concentration comprise entre 1 mg/L et 200 mg/L, et plus préférentiellement encore entre 10 mg/L et 50 mg/L ;
- ledit milieu de culture comprend en outre, à titre d'agents sélectifs :
   - de la L-ornithine, à une concentration comprise entre 15 g/L et 20 g/L,
   - du désoxycholate de sodium, à une concentration comprise entre de l'ordre de 1 g/L et de l'ordre de 10 g/L, et
   - éventuellement, du tergitol 4, à une concentration comprise entre de l'ordre de 0,5 mL/L et de l'ordre de 5 mL/L ;
- ledit milieu de culture est un milieu discriminatif, comprenant au moins une composante chromogénique choisie parmi :
   - un indicateur coloré de pH (par exemple, le rouge neutre, le rouge de phénol, le pourpre de bromocrésol, le couple bleu de bromothymol et fuchsine acide) apte à réaliser un marquage des colonies de microorganismes fermentaires d'au moins un des glucides présents dans le milieu,
   - un système thiosulfate de sodium / citrate de fer III, permettant la mise en évidence des bactéries productrices de H₂S,
   - des substrats chromogènes et/ou fluorogènes permettant la mise en évidence d'activités enzymatiques particulières ;
- ledit milieu de culture comprend une composante nutritive reprenant celle d'un milieu de culture choisi parmi :
   - la Gélose XLD (bioMérieux, France),
   - la Gélose chromID^{®} *Salmonella* Elite (bioMérieux, France),
   - la Gélose *Salmonella-Shigella* (bioMérieux, France) ;
- ledit milieu de culture comprend une composante nutritive et une composante sélective reprenant celles de la Gélose XLD, et est complémenté avec :
   - de la L-ornithine, à une concentration comprise entre 15 g/L et 20 g/L,
   - éventuellement, le X-*beta*-ribofuranoside, à une concentration comprise entre de l'ordre de 10 mg/L et de l'ordre de 200 mg/L,
   - éventuellement, un agent opacifiant tel que le dioxide de titane (TiO₂), notamment à une concentration comprise entre de l'ordre de 0,1 g/L et de l'ordre de 5 g/L, et
   - éventuellement, du tergitol 4, à une concentration comprise entre de l'ordre de 0,5 mL/L et de l'ordre de 5 mL/L.

L'invention s'étend également à une méthode d'inhibition de *Escherichia coli* dans laquelle de la L-ornithine est utilisée à une concentration comprise entre 15 g/L et 20 g/L. L'invention concerne également l'utilisation de la L-ornithine à une concentration supérieure à 10 g/L à des fins d'inhibition de la croissance et/ou du développement de *Escherichia coli.*

L'invention concerne également une méthode, un procédé d'enrichissement et de culture sélective de bactéries appartenant aux genres *Salmonella* et/ou *Shigella,* une utilisation de la L-ornithine à des fins de culture et/ou d'isolement de ces bactéries, ainsi qu'un milieu de culture, caractérisés par tout ou partie des caractéristiques techniques exposées ci-dessus et ci-dessous.

D'autres buts, caractéristiques et avantages de l'invention apparaîtront au vu de la description qui va suivre et des exemples développés ci-dessous, qui ont pour but de faciliter la compréhension de l'invention et de sa mise en œuvre. Ces exemples sont donnés à titre explicatif et ne sauraient limiter la portée de l'invention.

### EXEMPLES : Elaboration et évaluation des milieux de culture selon l'invention

### 1/ - Milieux de sélection selon l'invention, préparés sur la base d'une composition de Gélose XLD

### a) Préparation des Milieu A1 et Milieu A2

A partir de la composition de la Gélose XLD (bioMérieux, France), un milieu d'isolement sélectif classiquement utilisé pour la recherche de *Salmonella* et de *Shigella,* trois milieux de culture améliorés ont été préparés. Leurs compositions sont les suivantes :
- Milieu T : composition de la Gélose XLD avec ajout d'un agent opacifiant (TiO₂ ; 0,5 g/L) ;
- Milieu A1: composition du milieu T, avec ajout de L-ornithine (5 g/L),
- Milieu A2: composition du milieu T, avec ajout de L-ornithine (10 g/L).

A titre de L-ornithine, on peut notamment citer celle produite et commercialisée par la société SIGMA-ALDRICH, Etats-Unis (réf. O2375).

A titre de L-ornithine, on peut notamment citer celle produite et commercialisée par la société SIGMA-ALDRICH, Etats-Unis (réf. O2375).

### b) Evaluation des milieux pour la détection de Salmonella et Shigella

Différentes espèces de *Salmonella* (huit sérotypes incluant *S. enteritidis,* S. Thyphimurium, S. Paratyphi A, B et C, S. Gallinarum, S. Virchow, S. Derby), de *Shigella* (quatre espèces incluant *S. sonnei, S. boydii, S. flexneri, S. dysenteriae),* ainsi que 15 autres souches de bactéries à Gram négatif (en l'occurrence des *E. coli,* et d'autres entérobactéries appartenant aux genres *Proteus, Citrobacter, Enterobacter* et *Pseudomonas, Acinetobacter),* toutes issues de la collection de la Demanderesse, ont été utilisées.

Les bactéries ont été mises en suspension dans de l'eau physiologique, puis ensemencées sur les différents milieux, selon la technique dite des « 4 quadrants ». Les colonies formées sur les géloses ont été examinées visuellement après 18-24 heures d'incubation à 37 °C.

Les résultats obtenus sont synthétisés dans le Tableau 1, ci-après. Les inhibitions observées concernent soit la taille des colonies, soit la densité de croissance des souches sur les géloses.

**Tableau 1**

| Milieu | ***Salmonella*** - ***Shigella** :* souches détectées / *souches* testées | **souches *non-Salmonella,* non*****-Shigella** :* souches inhibées / souches testées |
|---|---|---|
| T | 16/16 | 0/15 |
| A1 | 16/16 | 7/15 |
| A2 | 16/16 | 7/15 |

Ces résultats montrent une bonne sensibilité de détection des souches de *Salmonella* et *Shigella* par tous les milieux. En revanche, seuls le Milieu A1 et le Milieu A2 permettent de diminuer la croissance des autres bactéries à Gram négatif, différentes de *Salmonella* et de *Shigella.*

### 2/ - Optimisation de la concentration en L-ornithine des milieux de sélection selon l'invention, préparés sur la base d'une composition de milieu XLD

### a) Préparation du Milieu A3

Au vu des résultats précédemment présentés, un nouveau milieu, noté Milieu A3, a été préparé. La composition de ce dernier reprend celle du Milieu A2, avec toutefois une concentration doublée en L-ornithine (20 g/L).

### b) Evaluation du Milieu A3 pour la détection de Salmonella et Shigella

Le Milieu A3 a été testé pour la détection de *Salmonella* et *Shigella,* et comparé au Milieu T et au Milieu A2. Pour ce faire, différentes espèces de *Salmonella* (sérotypes incluant *S. enteritidis, S.* Thyphimurium, S. Paratyphi A, B et C, S. Gallinarum, S. Virchow, S. Derby) de *Shigella* (espèces incluant *S. sonnei, S. boydii, S. flexneri, S. dysenteriae),* différentes souches *d'Escherichia coli* (8 souches), ainsi que d'autres bactéries à Gram négatif (*Proteus, Enterobacter et Pseudomonas*), toutes issues de la collection de la Demanderesse, ont été utilisées.

Les bactéries ont été mises en suspension dans de l'eau physiologique, puis ensemencées sur les milieux, selon la technique des 4 quadrants. Les colonies formées sur les géloses ont été examinées visuellement après 18-24 heures d'incubation à 37 °C.

Les résultats obtenus sont synthétisés dans le Tableau 2, ci-après. Les inhibitions observées concernent soit la taille des colonies, soit la densité de croissance des souches sur les géloses.

**Tableau 2**

| Milieu | ***Salmonella*** - ***Shigella** :* souches détectées / *souches* testées | ***Escherichia coli** :* souches inhibées / souches testées | **souches** ***non-Salmonella**,* non***-Shigella**,* **non-*E***. ***coli**:* souches inhibées / souches testées |
|---|---|---|---|
| T | 14/14 | 0/8 | 0/14 |
| A2 | 14/14 | 5/8 | 6/14 |
| A3 | 14/14 | 7/8 | 8/14 |

Ces résultats montrent une excellente sensibilité de tous les milieux pour la recherche des souches cibles de *Salmonella* et de *Shigella,* avec la détection de la totalité des quatorze souches pour les trois formules.

En revanche, seuls le Milieu A2 et le Milieu A3 permettent de diminuer la croissance des genres bactériens autres que *Salmonella* et *Shigella* et ce, notamment pour l'espèce *E. coli.* La meilleure sélectivité est obtenue à une concentration en L-ornithine, de 20 g/L.

### 3/ - Milieux de sélection selon l'invention, préparés sur la base d'une composition de Gélose chromID^{®} Salmonella Elite

### a) Préparation du Milieu B

Le Milieu B a été préparé à partir de la composition de la Gélose chromID^{®} *Salmonella* Elite (bioMérieux, France), à laquelle de la L-ornithine a été ajoutée pour obtenir une concentration finale de 15 g/L. La Gélose chromID^{®} *Salmonella* Elite est un milieu chromogénique initialement conçu pour l'isolement sélectif et l'identification de *Salmonella,* à partir de prélèvements d'origine humaine.

### b) Evaluation du Milieu B pour la détection de Salmonella et Shigella

Le Milieu B a été testé pour la détection de *Salmonella* et *Shigella,* et comparé au Milieu T, tel que précédemment décrit, ainsi qu'au Milieu A4. La composition de ce dernier reprend celle du Milieu T, à laquelle de la L-ornithine a été ajoutée pour obtenir une concentration finale de 15 g/L.

Pour cette évaluation, différents sérotypes de *Salmonella* incluant *S. enteritidis, S.* Thyphimurium, S. Paratyphi A, B et C, S. Gallinarum, S. Virchow, S. Derby), de *Shigella* incluant les espèces *S. sonnei, S. boydii, S.flexneri, S. dysenteriae),* différentes souches *d'Escherichia coli* (6 souches), ainsi que d'autres bactéries à Gram négatif (*Proteus, Enterobacter, Citrobacter, Pseudomonas*), des bactéries à Gram positif (staphylocoques, enterocoques) et des levures (*Candida*), toutes issues de la collection de la Demanderesse, ont été utilisées.

Les bactéries ont été mises en suspension dans de l'eau physiologique, puis ensemencées sur les milieux, selon la technique des 4 quadrants. Les colonies formées sur les géloses ont été examinées visuellement après 18-24 heures d'incubation à 37 °C.

Les résultats obtenus sont synthétisés dans le Tableau 3, ci-après. Les inhibitions observées concernent soit la taille des colonies, soit l'intensité de croissance des souches sur les géloses.

**Tableau 3**

| Milieu | ***Salmonella*** - ***Shigella** :* souches détectées / *souches* testées | ***Escherichia coli**:* souches inhibées / souches testées | **souches *non-Salmonella,* non*-Shigella,* non-*E***. ***coli**:* souches inhibées / souches testées |
|---|---|---|---|
| T | 16/16 | 0/6 | 4/19 |
| A4 | 16/16 | 5/6 | 10/19 |
| B | 13/16 | 6/6 | 17/19 |

Ces résultats montrent une excellente sensibilité du Milieu T et du Milieu A4 pour la recherche des souches de *Salmonella* et de *Shigella,* avec la détection de la totalité des seize souches testées. Le Milieu B permet de détecter treize souches sur les seize testées et montre une sélectivité imparfaite à l'égard de *Salmonella* et de *Shigella.*

En revanche, seuls le Milieu A4 et le Milieu B permettent de diminuer la croissance des microorganismes différents de *Salmonella* et *Shigella,* notamment celle de l'espèce *E. coli.*

### 4/ - Optimisation du pouvoir discriminant et chromogénique des milieux de sélection selon l'invention

### a) Préparation du Milieu C

Le Milieu C a été préparé à partir de la composition de la Gélose XLD (bioMérieux, France), à laquelle il a été rajouté un agent opacifiant (TiO₂ ; 0,5 g/L), de la L-ornithine (15 g/L) et un substrat chromogène, le X-*beta*-ribofuranoside (0,06 g/L).

### b) Evaluation du Milieu C pour la détection de Salmonella et Shigella

Le Milieu C a été testé pour la détection de *Salmonella* et *Shigella,* et comparé avec la Gélose XLD.

Pour cette évaluation, trente souches de *Salmonella* (sérotypes incluant *S. enteritidis, S.* Thyphimurium, S. Paratyphi A, B et C, S. Gallinarum, S. Virchow, S. Derby, S. Dublin, S. Choleraesuis, S. Infantis, S. Arizonae, S. Cubana), quarante souches de *Shigella* (espèces incluant *S. sonnei, S. boydii, S.flexneri, S. dysenteriae*), et quinze autres bactéries à Gram-négatif (*Proteus, Citrobacter, Enterobacter, E. coli* et *Pseudomonas, Acinetobacter*), toutes issues de la collection de la Demanderesse, ont été mises en suspension dans de l'eau physiologique, puis ensemencées sur les milieux, selon la technique des 4 quadrants. Les colonies formées sur les géloses ont été examinées visuellement après 18-24 heures d'incubation à 37 °C.

Les observations recueillies sont synthétisées dans le Tableau 4, ci-après.

**Tableau 4**

| Souches (nbre de souches testées) | Gélose XLD (souches détectées/souches testées) | | Milieu C (souches détectées/souches testées) | |
|---|---|---|---|---|
| ***Salmonella*** (30) | 30/30 (dont 28 incolores à centre noir) | colonies de 2 à 4 mm | 30/30 (dont 28 incolores à centre noir) | colonies de 1,5 à 3 mm |
| ***Shigella*** (40) | 40/40 (incolores) | colonies de 1,5 à 5 mm | 40/40 (colorées en gris-bleu) | colonies de 1 à 4 mm (4 souches avec une densité de croissance plus faible) |
| Autres souches à Gram négatif (15) | 15/15 (densité de croissance importante) | colonies de 2 à 5 mm | 10/15 | Observation des inhibitions, basée sur la taille des colonies, la densité de croissance |

Ces résultats montrent une excellente sensibilité de la Gélose XLD et du Milieu C, pour la recherche des souches de *Salmonella* et de *Shigella,* avec la détection de la totalité des soixante-dix souches.

En revanche, seul le Milieu C permet de diminuer la croissance des autres bactéries à Gram négatif.

### 5/ - Optimisation du pouvoir sélectif des milieux de sélection selon l'invention

### a) Préparation du Milieu D

Le Milieu D a été préparé à partir de la composition de la Gélose XLD (bioMérieux, France), à laquelle il a été rajouté un agent opacifiant (TiO₂ ; 0,5 g/L), de la L-ornithine (15 g/L), un substrat chromogène, le X-*beta*-ribofuranoside (0,06 g/L) et du tergitol4 (1,5 mL/L).

Le tergitol 4 utilisé peut être notamment celui fabriqué et commercialisé par SIGMA-ALDRICH, Etats-Unis (réf. Niaproof 4 N1404).

### b) Evaluation du Milieu D pour la détection de Salmonella et Shigella

Le Milieu D a été testé pour la détection de *Salmonella* et *Shigella,* et comparé avec la Gélose XLD.

Pour cette évaluation, ont été utilisées :
- 19 souches de *Salmonella* (3 de S. Typhimurium, 2 de *S. enteritidis,* 1 de S. Virchow, 3 de S. Paratyphi A, 3 de S. Paratyphi B, 2 de S. Paratyphi C, 1 de *S*. Cubana, 1 de S. Dublin, 1 de S. Arizonae, 1 de S. Gallinarum, 1 de S. Choleraesuis);
- 29 souches de *Shigella* (4 de *S. boydii,* 13 de *S.flexneri,* 12 de *S. sonnei*),
- 15 souches de bactéries à Gram négatif, non *Salmonella* ni *Shigella* (4 de *E. coli, 2* de *P. vulgaris,* 1 de *P. mirabilis,* 1 de *E. cloacae,* 1 de *E. aerogenes,* 1 de *P. aeruginosa,* 1 de *A. baumanii,* 1 de *E. hoshinae,* 2 de C. *braakii,* 1 de *C. youngae).*

Ces souches, issues de la de la collection de la Demanderesse, ont été mises en suspension dans de l'eau physiologique, puis ensemencées sur les milieux, selon la technique des 4 quadrants. Les colonies formées sur les géloses ont été examinées visuellement après 18-24 heures d'incubation à 37 °C.

Le Tableau 5, ci-après, rassemble les observations recueillies pour des cultures de souches pures.

**Tableau 5**

| Souches (nbre de souches testées) | Gélose XLD (souches détectées/souches testées) | | Milieu C (souches détectées/souches testées) | |
|---|---|---|---|---|
| *Salmonella* (19) | 19/19 (incolores à centre noir) | 15 souches formant des colonies de 2 mm et plus ; 4 souches formant des colonies de moins de 2 mm | 19/19 (17 incolores à centre noir, 2 gris-bleu) | 4 souches formant des colonies de 2 mm et plus ; 15 souches formant des colonies de moins de 2 mm |
| *Shigella* (29) | 29/29 (toutes incolores) | 20 souches formant des colonies de 2 mm et plus ; 9 souches formant des colonies de moins de 2 mm | 29/29 (28 colorées en gris-bleu, 1 incolore) | 10 souches formant des colonies de 2 mm et plus ; 19 souches formant des colonies de moins de 2 mm |
| Autres souches à Gram négatif (15) | 15/15 | 9 souches formant des colonies de 2 mm et plus ; 9 souches formant des colonies de moins de 2 mm | 15/15 | 12 souches pour lesquelles une réduction notable de la taille des colonies est observée |

Le Tableau 6, ci-après, rassemble les observations recueillies pour la culture de souches en mélange.

**Tableau 6**

| | Gélose XLD | | | Milieu C | | |
|---|---|---|---|---|---|---|
| | Fertilité du milieu | Taille | Activité enzymatique | Densité de croissance | Taille | Activité enzymatique |
| S. *typhimurium* (ATCC 14028) + C. *braaki* (ATCC15580) | 2,2 | 2 mm | colonies incolores à centre noir | 2,2 | 1,5 mm | colonies incolores à centre noir |
| | 2,2 | 1,5 mm | Colonies jaunes | 2,2 | 1,5 mm | colonies jaune-vert |
| S. *enteritidis* (IM195) + *Sh. boydii* (ATCC 8700) | 2,3 | 2,5 mm | colonies incolores à centre noir | 2,3 | 1,5 mm | colonies incolores à centre noir |
| | 2,1 | 0,75 mm | colonies incolores | 2,3 | 0,5-0,75 mm | colonies gris-bleu |
| *S*. *paratyphi* B (ATCC 10719) + *E. coli* (ATCC 25922) | 2,3 | 2,5 mm | colonies incolores à centre noir | 2,3 | 2 | colonies incolores à centre noir |
| | 2,1 | 1-1,5 mm | jaune | 1,1 | colonies très fines | colonies incolores |
| *S*. *paratyphi* C (ATCC 13428) + *E. coli* (ATCC 12453) | 2,1 | 1,5-2 mm | colonies incolores à centre noir | 3,1 | 1,5 mm | colonies incolores à centre noir |
| | 2,2 | 2,5-3 mm | colonies légèrement beiges | 3,1 | 2 mm | colonies beiges |

## Revendications

1. Procédé d'enrichissement et de culture sélective de bactéries appartenant aux genres *Salmonella* et/ou *Shigella* contenues dans un échantillon biologique, dans lequel on ensemence tout ou partie dudit échantillon dans/sur un milieu de culture comprenant des éléments nutritifs propices au développement et à la croissance desdites bactéries, **caractérisé en ce que** ledit milieu de culture comprend également de la L-ornithine, à titre d'agent sélectif, à une concentration comprise entre 15 g/L et 20 g/L.

2. Procédé selon la revendication 1, **caractérisé en ce que** ledit milieu de culture comprend au moins un agent sélectif additionnel choisi parmi : le désoxycholate de sodium, le tergitol 4, des sels biliaires, le violet cristal et le vert brillant.

3. Procédé selon la revendication 1 ou 2, **caractérisé en ce que** ledit milieu de culture comprend à titre d'agents sélectifs :
- de la L-ornithine, à une concentration comprise entre 15 g/L et 20 g/L, et
- du désoxycholate de sodium, à une concentration comprise entre de l'ordre de 1 g/L et de l'ordre de 10 g/L.

4. Procédé selon la revendication 3, **caractérisé en ce que** ledit milieu de culture comprend également, à titre d'agent sélectif, du tergitol 4, à une concentration comprise de l'ordre de 0,5 mL/L et de l'ordre de 5 mL/L.

5. Procédé selon l'une quelconque des revendications précédentes, **caractérisé en ce que** ledit milieu de culture comprend au moins une composante chromogénique choisie parmi :
- un indicateur coloré de pH apte à réaliser le marquage des colonies de microorganismes fermentaires d'au moins un des glucides présents dans le milieu ;
- un système thiosulfate de sodium / citrate de fer III, permettant la mise en évidence des bactéries productrices de H₂S ;
- des substrats chromogènes et/ou fluorogènes permettant la mise en évidence d'activités enzymatiques particulières.

6. Procédé selon l'une quelconque des revendications 1 à 5, **caractérisé en ce que** ledit milieu de culture comprend les éléments nutritifs d'un milieu de culture choisi parmi :
- la Gélose XLD,
- la Gélose *Salmonella-Shigella.*

7. Procédé selon la revendication 5, **caractérisé en ce que** ledit milieu de culture comprend les éléments nutritifs et une composante sélective de la Gélose XLD,

8. Milieu de culture comprenant des éléments nutritifs propices au développement et à la croissance de bactéries appartenant aux genres *Salmonella* et *Shigella,* **caractérisé en ce qu'**il comprend également de la L-ornithine, à une concentration comprise entre 15 g/L et 20 g/L.

9. Milieu de culture selon la revendication 8, **caractérisé en ce qu'**il comprend au moins un agent sélectif additionnel choisi parmi le désoxycholate de sodium, le tergitol 4, des sels biliaires, le violet cristal et le vert brillant.

10. Milieu de culture selon la revendication 8 ou 9, **caractérisé en ce qu'**il comprend à titre d'agents sélectifs :
- de la L-ornithine, à une concentration comprise entre 15 g/L et 20 g/L, et
- du désoxycholate de sodium, à une concentration comprise entre de l'ordre de 1 g/L et de l'ordre de 10 g/L.

11. Milieu de culture selon l'une quelconque des revendications 8 à 10, **caractérisé en ce qu'**il comprend les éléments nutritifs d'un milieu de culture choisi parmi :
- la Gélose XLD,
- la Gélose *Salmonella-Shigella.*

12. Milieu de culture selon l'une quelconque des revendications 8 à 11, **caractérisé en ce qu'**il comprend les éléments nutritifs et une composante sélective de la Gélose XLD.

## Patentansprüche

1. Verfahren zum Anreichern und selektiven Anzüchten von Bakterien, die den Gattungen *Salmonella* und/oder *Shigella* angehören, wobei sie in einer biologischen Probe enthalten sind, wobei im Rahmen desselben die Gesamtheit oder eine Teilmenge der Probe in/auf ein Kulturmedium überimpft wird, das Nährelemente umfasst, welche die Entwicklung und das Wachstum dieser Bakterien begünstigen, **dadurch gekennzeichnet, dass** das Kulturmedium weiterhin L-Ornithin, als Selektionsmittel, in einer Konzentration im Bereich von 15 g/L bis 20 g/L umfasst.

2. Verfahren nach Anspruch 1, **dadurch gekennzeichnet, dass** das Kulturmedium mindestens ein zusätzliches Selektionsmittel umfasst, das aus den folgenden ausgewählt ist: Natriumdesoxycholat, Tergitol 4, Gallensalze, Kristallviolett und Brillantgrün.

3. Verfahren nach Anspruch 1 oder 2, **dadurch gekennzeichnet, dass** das Kulturmedium als Selektionsmittel die folgenden umfasst:
- L-Ornithin, in einer Konzentration im Bereich von 15 g/L bis 20 g/L, und
- Natriumdesoxycholat, in einer Konzentration im Bereich einer Größenordnung von 1 g/L bis zu einer Größenordnung von 10 g/L.

4. Verfahren nach Anspruch 3, **dadurch gekennzeichnet, dass** das Kulturmedium weiterhin, als Selektionsmittel, Tergitol 4 in einer Konzentration im Bereich einer Größenordnung von 0,5 mL/L bis zu einer Größenordnung von 5 mL/L umfasst.

5. Verfahren nach einem beliebigen der vorhergehenden Ansprüche, **dadurch gekennzeichnet, dass** das Kulturmedium mindestens einen chromogenen Bestandteil umfasst, der aus den folgenden ausgewählt ist:
- einem pH-Farbindikator, der dafür geeignet ist, die Markierung von Mikroorganismenkolonien durchzuführen, welche mindestens eines der Kohlenhydrate fermentieren, wie sie in dem Medium vorliegen;
- einem Natriumthiosulfat/Eisen(III)citrat-System, mittels welchem Bakterien nachgewiesen werden können, die H₂S produzieren;
- chromogenen und/oder fluorogenen Substraten, mittels derer besonderer Enzymaktivitäten nachgewiesen werden können.

6. Verfahren nach einem beliebigen der Ansprüche 1 bis 5, **dadurch gekennzeichnet, dass** das Kulturmedium die Nährelemente eines Kulturmediums umfasst, welches aus den folgenden ausgewählt ist:
- XLD-Agar
- *Salmonella-Shigella-*Agar*.*

7. Verfahren nach Anspruch 5, **dadurch gekennzeichnet, dass** das Kulturmedium die Nährelemente und einen selektiv wirkenden Bestandteil von XLD-Agar umfasst.

8. Kulturmedium, das Nährelemente umfasst, welche die Entwicklung und das Wachstum von Bakterien begünstigen, die den Gattungen *Salmonella* und *Shigella* angehören, **dadurch gekennzeichnet, dass** es weiterhin L-Ornithin in einer Konzentration im Bereich von 15 g/L bis 20 g/L umfasst.

9. Kulturmedium nach Anspruch 8, **dadurch gekennzeichnet, dass** es mindestens ein zusätzliches Selektionsmittel umfasst, welches aus Natriumdesoxycholat, Tergitol 4, Gallensalzen, Kristallviolett und Brillantgrün ausgewählt ist.

10. Kulturmedium nach Anspruch 8 oder 9, **dadurch gekennzeichnet, dass** es als Selektionsmittel die folgenden umfasst:
- L-Ornithin, in einer Konzentration im Bereich von 15 g/L bis 20 g/L, und
- Natriumdesoxycholat, in einer Konzentration im Bereich einer Größenordnung von 1 g/L bis zu einer Größenordnung von 10 g/L.

11. Kulturmedium nach einem beliebigen der Ansprüche 8 bis 10, **dadurch gekennzeichnet, dass** es die Nährelemente eines Kulturmediums umfasst, welches aus den folgenden ausgewählt ist:
- XLD-Agar
- und
- *Salmonella-Shigella-*Agar*.*

12. Kulturmedium nach einem beliebigen der Ansprüche 8 bis 11, **dadurch gekennzeichnet, dass** es die Nährelemente und einen selektiv wirkenden Bestandteil von XLD-Agar umfasst.

## Claims

1. A method for enrichment and selective culture of bacteria of the genera *Salmonella* and/or *Shigella* contained in a biological sample, in which a part or the whole of said sample is seeded in/on a culture medium comprising a nutrient component that favors the development and growth of said bacteria, **characterized in that** said culture medium also comprises L-ornithine, as a selective agent, at a concentration between 15 g/L and 20 g/L.

2. The method as claimed in claim 1, **characterized in that** said culture medium comprises at least one additional selective agent selected from: sodium deoxycholate, Tergitol 4, bile salts, crystal violet and brilliant green.

3. The method as claimed in 1 or 2, **characterized in that** said culture medium comprises as selective agents:
- L-ornithine, at a concentration between 15 g/L and 20 g/L, and
- sodium deoxycholate, at a concentration between about 1 g/L and about 10 g/L.

4. The method as claimed in claim 3, **characterized in that** said culture medium also comprises, as selective agent, Tergitol 4, at a concentration between about 0.5 mL/L and about 5 mL/L.

5. The method as claimed in any one of preceding claims, **characterized in that** said culture medium comprises at least one chromogenic component selected from:
- a pH color indicator able to perform labeling of the colonies of microorganisms fermenting at least one of the carbohydrates present in the medium;
- a sodium thiosulfate / iron(III) citrate system, allowing detection of the H₂S-producing bacteria;
- chromogenic and/or fluorogenic substrates allowing the detection of particular enzyme activities.

6. The method as claimed in any one of claims 1 to 5, **characterized in that** said culture medium comprises the nutrient components repeating that of a culture medium selected from:
- XLD Agar, and
- *Salmonella-Shigella* Agar.

7. The method as claimed in claim 5, **characterized in that** said culture medium comprises the nutrient components and a selective component repeating those of XLD Agar.

8. A culture medium comprising a nutrient component that favors the development and growth of bacteria of the genera *Salmonella* and *Shigella,* **characterized in that** it also comprises L-ornithine, as a selective agent, at a concentration between 15 g/L and 20 g/L.

9. The culture medium as claimed in claim 8, **characterized in that** it comprises at least one additional selective agent selected from sodium deoxycholate, Tergitol 4, bile salts, crystal violet and brilliant green.

10. The culture medium as claimed in any one of claims 10 to 12, **characterized in that** it comprises as selective agents:
- L-ornithine, at a concentration between 15 g/L and 20 g/L, and
- sodium deoxycholate, at a concentration between about 1 g/L and about 10 g/L.

11. The culture medium as claimed in any one of claims 8 to 10, **characterized in that** it comprises the nutrient components repeating that of a culture medium selected from:
- XLD Agar, and
- *Salmonella-Shigella* Agar.

12. The culture medium as claimed in any one of claims 8 to 11, **characterized in that** it comprises the nutrient components and a selective component repeating those of XLD Agar.
